# EUROPEAN PATENT APPLICATION

(11) **EP 3 736 774 A1**
(43) Date of publication of application: **11.11.2020**
(21) Application number: 20170582.9
(22) Date of filing: 21.04.2020
(51) Int. Cl.: G06T 11/20, G16H 50/20

(54) **RULE PRESENTATION METHOD, STORAGE MEDIUM, AND RULE PRESENTATION APPARATUS**

(30) Priority: 09.05.2019 JP 2019089288
(71) Applicant: FUJITSU LIMITED, Kanagawa 211-8588 (JP)
(72) Inventor: Kobayashi, Ken, Kanagawa, 211-8588 (JP); Katoh, Takashi, Kanagawa, 211-8588 (JP); Ura, Akira, Kanagawa, 211-8588 (JP)
(74) Representative: Hoffmann Eitle

(57) **Abstract**

A rule presentation method by a computer, includes acquiring training data that is a set of rules in which a combination of attributes is associated with one of a positive example and a negative example; extracting a plurality of rules that specify one of a positive example and a negative example according to the number of positive examples and the number of negative examples for one or more combinations of attributes, based on the acquired training data; acquiring first data that has a combination of attributes different from the combination of attributes included in the training data and is not associated with a label that designates the positive example or the negative example; selecting a rule related to the combination of attributes included in the first data from among the plurality of specified rules; generating second data in which a label different from the label of the positive example or the negative example specified by the selected rule is associated with the first data; specifying the number of samples of the first data in which the label of the positive example or the negative example specified by the selected rule changes, based on the generated second data; and determining an order of rules to be presented based on the number of samples.

## Description

### FIELD

The embodiment discussed herein is related to a rule presentation method and the like.

### BACKGROUND

When machine learning or the like is used to support judgment of a user, it is demanded to output rules and hypotheses in a form that may be directly understood by the user. For example, when performing medical treatment support for a doctor, it is desirable to make a final judgment of the medical treatment in consideration of not only a single prediction result but also an alternative prediction, and a rule that leads to the alternative prediction for a certain input (attribute of a medical treatment subject. In the following description, a medical treatment subject is simply referred to as a "subject".

In the related art, when an input condition corresponds to a plurality of rules, all of the plurality of corresponding rules are listed.

International Publication Pamphlet No. WO 2017/081715, International Publication Pamphlet No. WO 2013/172310, Japanese Laid-open Patent Publication No. 6-102907, and Japanese Laid-open Patent Publication No. 2016-212825 are examples of related art.

### SUMMARY

### [TECHNICAL PROBLEM]

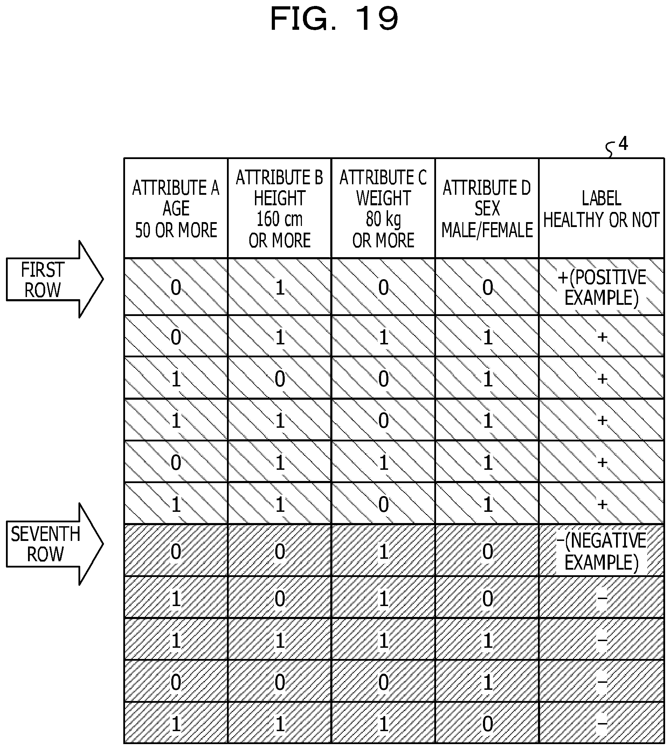

FIG. 19 is a diagram illustrating an example of training data used in the related art. As illustrated in FIG. 19, training data 4 associates a plurality of attributes with a label. The attribute corresponds to an attribute of a subject, and includes, for example, an attribute A, an attribute B, an attribute C, and an attribute D.

The attribute A is "indicating whether or not age is 50 (age) or more", and when the age of the subject is 50 or more, a value becomes "1", and when the age of the subject is less than 50, the value becomes "0".

The attribute B is "indicating whether or not height is 160 cm or more ", and when the height of the subject is 160 cm or more, the value becomes "1", and when the height of the subject is less than 160 cm, the value becomes "0".

The attribute C is "indicating whether or not weight is 80 kg or more", and when the weight of the subject is 80 kg or more, the value becomes "1", and when the weight of the subject is less than 80 kg, the value becomes "0".

The attribute D is "indicating whether sex is male or female ", and when the sex of the subject is male, the value becomes "1", and when the sex of the subject is female, the value becomes "0".

The label is that a value corresponding to each attribute of a record is "indicating whether or not it is healthy, the value becomes "+ (positive example)" when it is healthy, and the value becomes "- (negative example)" when it is not healthy. For example, in the record of the first row, when the attribute A is "0", the attribute B is "1", the attribute C is "0", and the attribute D is "0", the label is "+". In the record of the seventh row, when the attribute A is "0", the attribute B is "0", the attribute C is "1", and the attribute D is "0", the label is "-".

In the related art, a set of rules is generated using training data 4 illustrated in FIG. 19, and all rules satisfying a given condition are listed using the set of rules. For example, when a condition "weight is less than 80 kg" is input, a "rule leading to a positive example" in which the attribute C is "0" is output. When a condition "female having weight of 80 kg or more" is input, a "rule leading to a negative example" in which the attribute C is "1" is output. When a condition "height is less than 160 cm and weight is 80 kg or more " is input, a "rule leading to a negative example" in which the attribute B is "0" and the attribute C is "1" is output.

However, in the related art described above, when the input condition corresponds to a plurality of rules, a plurality of the corresponding rules are listed, and thus it is difficult for the user to select a desired rule from the plurality of listed rules.

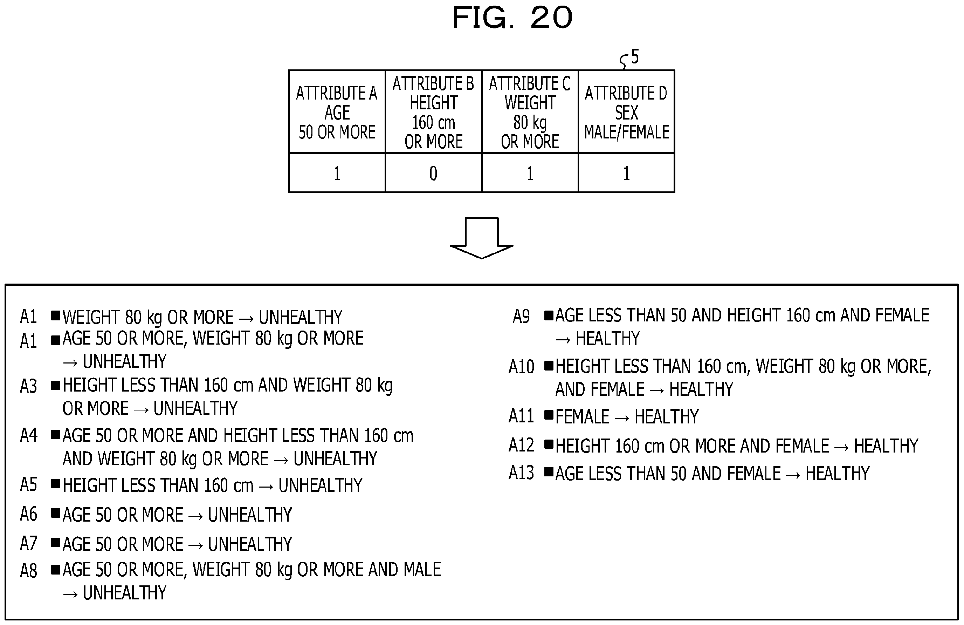

FIG. 20 is a diagram for describing a problem of the related art. For example, it is assumed that the condition of the subject is "age 50 or more, height less than 160 cm, weight 80 kg or more, and male". In the related art, a plurality of rule A1 to A13 corresponding to condition 5 are listed by comparing the condition 5 of the subject with a set of rules based on training data. Even if these rules A1 to A13 are presented at a time, it is difficult for the user to select a desired rule.

For example, the rule A1 is a rule indicating that "it is not healthy (unhealthy) when weight is 80 kg or more ", and is a rule corresponding to the condition 5. Although the description regarding the rules A2 to A13 is omitted, all of the rules A2 to A13 are the rules corresponding to the condition 5.

It is also conceivable to calculate a ratio of the number of samples supporting a rule in a set of possible rules to the number of samples included in the rule as a correct answer rate of the corresponding rule, and to present only the rule whose correct answer rate exceeds a threshold value among the rules corresponding to the condition. However, it is difficult to set an appropriate threshold value, and a lower threshold value that includes a relatively large number of rules is set, and thus, it is also difficult to narrow down the rules.

In one aspect, an object of the embodiment is to provide a rule presentation method, a computer-readable recording medium, and a rule presentation apparatus that allow a user to select a desired rule from a plurality of rules corresponding to a condition.

### [SOLUTION TO PROBLEM]

According to an aspect of the embodiments, an apparatus includes acquiring training data that is a set of rules in which a combination of attributes is associated with one of a positive example and a negative example; specifying a plurality of rules that specify one of a positive example and a negative example according to the number of positive examples and the number of negative examples for one or more combinations of attributes, based on the acquired training data; acquiring first data that has a combination of attributes different from the combination of attributes included in the training data and is not associated with a label that designates the positive example or the negative example; selecting a rule related to the combination of attributes included in the first data from among the plurality of specified rules; generating second data in which a label different from the label of the positive example or the negative example specified by the selected rule is associated with the first data; specifying the number of samples of the first data in which the label of the positive example or the negative example specified by the selected rule changes, based on the generated second data; and determining an order of rules to be presented based on the number of samples.

### [ADVANTAGEOUS EFFECTS OF INVENTION]

According to an embodiment, a desired rule from a plurality of conditions corresponding to the conditions may be selected.

### BRIEF DESCRIPTION OF DRAWINGS

FIG. 1 is a diagram (1) for describing a Karnaugh map;
FIG. 2 is a diagram (2) for describing a Karnaugh map;
FIG. 3 is a diagram (3) for describing a Karnaugh map;
FIG. 4 is a diagram for describing processing of a rule presentation apparatus according to an example;
FIG. 5 is a graph illustrating a relationship between a correct answer rate and the number of samples;
FIG. 6 is a graph illustrating a relationship between a correct answer rate of a rule corresponding to a designated attribute and the number of samples;
FIG. 7 is a functional block diagram illustrating a configuration of a rule presentation apparatus according to the example;
FIG. 8 is a diagram illustrating an example of a data structure of training data;
FIG. 9 is a diagram illustrating an example of designated condition data;
FIG. 10 is a diagram illustrating an example of rule set data;
FIG. 11 is a diagram illustrating an example of presentation candidate set data;
FIG. 12 is a diagram for describing processing of a specifying unit;
FIG. 13 is a diagram (1) for describing processing of a determination unit;
FIG. 14 is a diagram (2) for describing processing of the determination unit;
FIG. 15 is a diagram (3) for describing processing of the determination unit;
FIG. 16 is a diagram illustrating an example of screen information generated by the determination unit;
FIGs. 17A and 17B are a flowchart illustrating a processing procedure of the rule presentation apparatus according to the example;
FIG. 18 is a diagram illustrating an example of a hardware configuration of a computer that realizes the same function as that of the rule presentation apparatus according to the example;
FIG. 19 is a diagram illustrating an example of training data used for machine learning in the related art; and
FIG. 20 is a diagram for describing a problem of the related art.

### DESCRIPTION OF EMBODIMENTS

Hereinafter, examples of the rule presentation method, the rule presentation program, and the rule presentation apparatus disclosed in the present specification will be described in detail with reference to the drawings. The present disclosure is not limited by the examples.

### Example

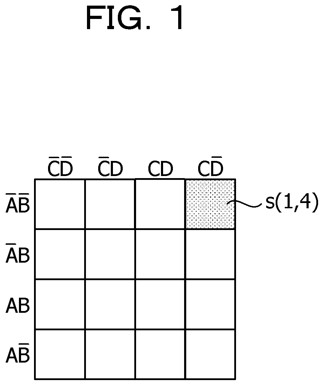

The Karnaugh map used in the example will be described. FIGs. 1, 2, and 3 are diagrams for describing a Karnaugh map. The Karnaugh map illustrates a logical expression. As an example, the Karnaugh maps illustrated in FIGs. 1 to 3 are Karnaugh maps of logical expressions using attributes A, B, C, and D as logical variables.

FIG. 1 will be described. The first row of the Karnaugh map is a row corresponding to "notA and notB". The second row is a row corresponding to "notA and B". The third row is a row corresponding to "A and B". The fourth row is a row corresponding to "A and notB".

The first column of the Karnaugh map is a row corresponding to "note and notD". The second row is a row corresponding to "notC and D". The third row is a row corresponding to "C and D". The fourth column is a row corresponding to "C and notD".

In this example, when indicating a cell in the n-th row and m-th column in the Karnaugh map, it is represented as s(n, m). For example, the cell in the first row and the fourth column is s(1, 4). s(1, 4) indicates that the attribute is "notA and notB and C and D".

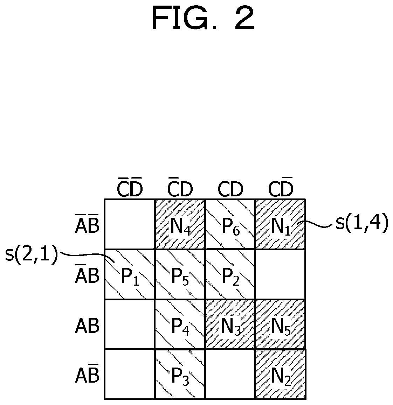

Description continues with reference to FIG. 2. The rule presentation apparatus of this example sets Pₙ (Positive) and Nₙ (Negative) in each cell of the Karnaugh map based on each sample (record) of training data. The suffix n is for the sake of convenience to distinguish each P and each N.

For example, when a sample corresponding to the cell of s(1, 4) is a negative example, the rule presentation apparatus according to this example sets "Ni" in the cell of s(1, 4). For example, a sample having the attribute "notA and notB and C and notD" is a negative example.

When the sample corresponding to the cell of s(2, 1) is a positive example, the rule presentation apparatus sets "Pi" in the cell of s(2, 1). For example, a sample having the attribute "notA and B and notC and notD" is a positive example.

Although the description is omitted, the other cells included in the Karnaugh map are set to "N" when the sample is negative example, and "P" is set when the sample is a positive example. When the corresponding sample is not present in the training data, nothing is set in the cell.

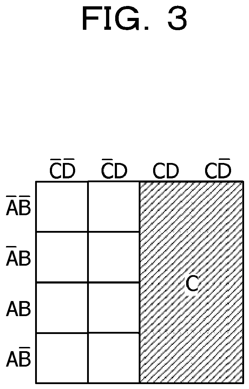

Description continues with reference to FIG. 3. In a Karnaugh map, a corresponding cell is determined according to a combination of attributes. As illustrated in FIG. 3, the cells corresponding to the attribute "C" become cells included in the third row and fourth row of the Karnaugh map. The rule presentation apparatus specifies a rule and a correct answer rate corresponding to the attribute "C" according to the number of the positive example and the negative examples included in the attribute "C".

For example, when the number of cells to which a positive example is assigned among the cells included in the attribute C is larger than the number of cells to which the negative example is assigned, the rule corresponding to the attribute C is a rule leading to the "positive example". The correct answer rate of such a rule is a percentage of the number of positive examples to the number of positive examples and the number of negative examples of the cells included in the attribute C.

In contrast, when the number of cells to which the positive example is assigned among the cells included in the attribute "C" is smaller than the number of cells to which the negative example is assigned, the rule corresponding to the attribute C is a rule leading to the "negative example". The correct answer rate of such a rule is a percentage of the number of negative examples to the number of positive examples and negative examples of the cells included in the attribute C.

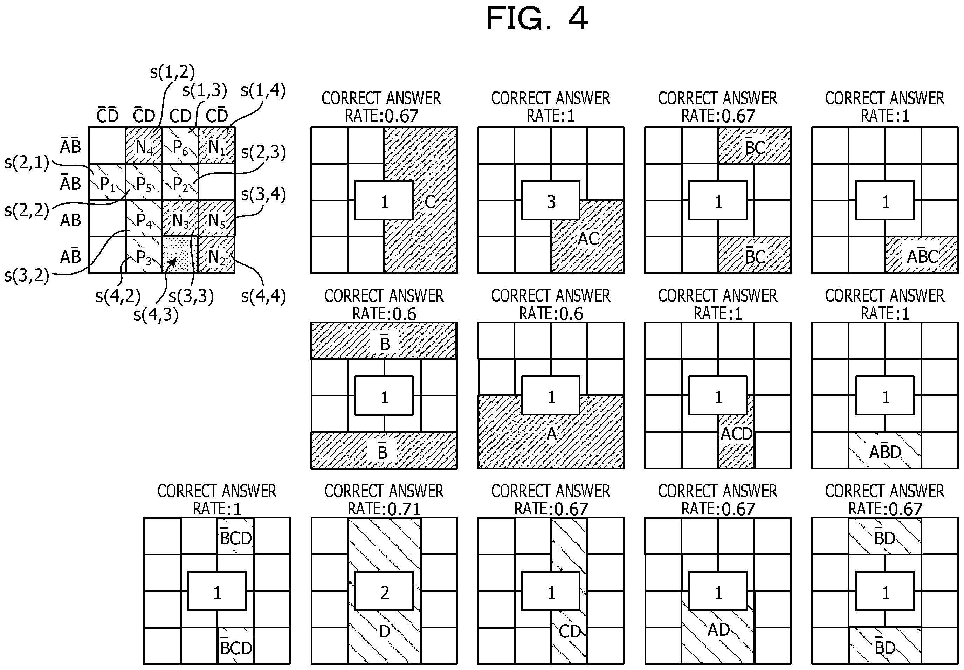

Next, an example of processing performed by the rule presentation apparatus according to this example will be described. FIG. 4 is a diagram for describing processing of the rule presentation apparatus according to this example. The rule presentation apparatus sets P or N to each cell in the Karnaugh map based on training data which is a set of combinations of attributes and rules that lead to the positive example or the negative example.

In the example illustrated in FIG. 4, the rule presentation apparatus sets N₁, N₂, N₃, N₄, and N₅ in the cells s(1, 4), s(4, 4), s(3, 3), s(1, 2), and s(3, 4) of the Karnaugh map, respectively. The rule presentation apparatus sets P₁, P₂, P₃, P₄, and P₅ in the cells s(2, 1), s(2, 3), s(4, 2), s(3, 2), s(2, 2), and s(1, 3) of the Karnaugh map, respectively.

For example, a case where the user designates a rule presentation request of "A and notB and C and D" as a condition of attribute to the rule presentation apparatus will be described. In the Karnaugh map, the cell corresponding to the condition of attribute "A and notB and C and D" is s(4, 3).

When designation of the condition of attribute "A and notB and C and D" is received, the rule presentation apparatus first specifies, from each of the samples of the training data, a plurality of rules, in which the correct answer rate is equal to or greater than the threshold value, among the rules leading to the positive example or the negative example of the one or more attributes. In this example, as an example, the threshold value of the correct answer rate is set to "0.6 (60%)". The rule presentation apparatus specifies rules related to the condition of attribute "A and notB and C and D" among the rules in which the correct answer rate is equal to or greater than the threshold value.

For example, the rules in which the correct answer rate is equal to or greater than the threshold value and which are related to the condition of attribute "A and notB and C and D" are the following rules. For example, the rule corresponding to the designated attribute includes a rule (correct answer rate:0.6) of attribute "A", a rule (correct answer rate:0.6) of attribute "notB", a rule (correct answer rate:0.67) of attribute "C", and a rule (correct answer rate:0.71) of attribute "D". The rule corresponding to the designated attribute is a rule (correct answer rate:0.67) of attribute "CD", a rule (correct answer rate:0.67) of attribute "notB and C", a rule (correct answer rate:0.67) of attribute "notB and D", a rule (correct answer rate:1) of attribute "A and C and D", a rule (correct answer rate:1) of attribute "A and notB and C", and a rule (correct answer rate:1) of the attribute "A and notB and D" and a rule (correct answer rate:1) of the attribute "notB and C and D".

Among the rules corresponding to the designated attributes, rules leading to the negative example are rules of the attributes "C, "AC", "notB and C", "A and notB and C", "notB", "A", and "ACD".

Among the rules corresponding to the designated attributes, rules leading to the positive example are rules of the attributes "A and notB and D", "notB and C and D", "D", "C and D", "A and D", and "notB and D".

Subsequently, the rule presentation apparatus calculates the "minimum number of samples" for a plurality of rules corresponding to the designated attribute. The rule presentation apparatus sets a label, which is opposite to a label led by the rule, for the label of the sample of the designated attribute, increases the number of samples, and calculates, first, the number of samples whose correct answer rate of the rule becomes less than the threshold value as the minimum number of samples. The rule with a larger minimum number of samples is less likely to fluctuate in the result led by the rule, and may be said to be a highly reliable rule.

For example, the minimum number of samples will be described using a rule that leads to the "negative example" of the attribute "C". The rule presentation apparatus sets the label of the sample of the cell s(3, 3) as the "positive example", and calculates, for the first time when the number of samples is reached a number, whether the correct answer rate of the rule is less than the threshold value. For example, when one sample leading to the "positive example" is added to the cell s(3, 3), the correct answer rate of the attribute "C" becomes less than the threshold value for the first time, and thus the minimum number of samples of the attribute "C" is "1".

The minimum number of samples will be described using a rule that leads to the "positive example" of the attribute "D". The rule presentation apparatus sets the label of the sample of the cell s(3, 3) as the "negative example", and calculates, for the first time when the number of samples is reached a number, whether the correct answer rate of the rule is less than the threshold value. For example, when two samples leading to the "negative example" are added to the cell s(3, 3), the correct answer rate of the attribute "D" becomes less than the threshold value, and thus the minimum number of samples of the attribute "D" is "2".

The rule presentation apparatus rearranges the rules among the rules corresponding to the designated attribute in descending order of the minimum number of samples, and presents each rule to the user according to the rearranged order. In the example illustrated in FIG. 4, first, the rule of the attribute "AC" is presented to the user, and second, the rule of the attribute "D" is presented to the user.

As described above, the rule presentation apparatus according to this example presents rules in an order in which the rule (rule with a large minimum number of samples) whose label is unlikely to change is prioritized even if_a label corresponding to the designated attribute is opposite to the corresponding rule among a plurality of rules related to the designated attribute. Thus, the rule desired by the user may be selected from a plurality of rules corresponding to the condition.

The rule with a large minimum number of samples may be said to be a score indicating reliability. Based on the minimum number of samples, the rule presentation apparatus may sequentially present rules in which the trade-off relationship between the correct answer rate and the number of samples is taken into consideration by ordering the rules, even if balance between the height of the correct answer rate of the rule and the number of samples included in the rule is not explicitly designated by the user.

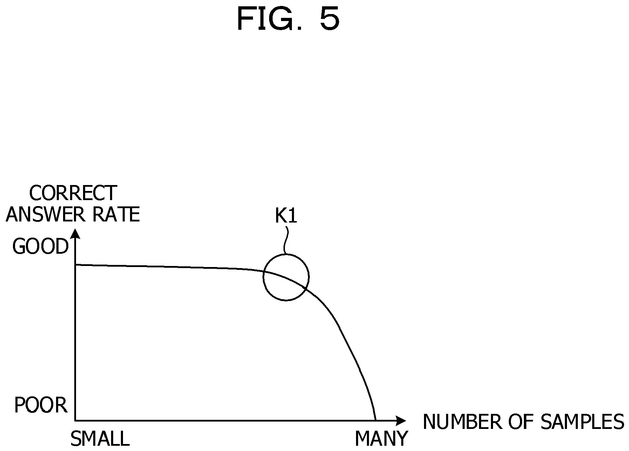

FIG. 5 is a graph illustrating the relationship between the correct answer rate and the number of samples. The vertical axis of the graph in FIG. 5 is an axis corresponding to the correct answer rate of the rule. The horizontal axis of the graph is an axis corresponding to the number of samples included in the rule. A hypothesis that a rule that is robust against data change has a large number of samples is established. As a result, the user wants to select a rule with a large number of samples if the correct answer rates of the rules are approximately the same. The user wants to select a rule with a high correct answer rate if the number of samples indicating the rule is approximately the same. Generally, although the correct rate of rules and the number of samples included in the rule are in a trade-off relationship, the rule presentation apparatus preferentially presents a rule whose relationship between the correct answer rate and the number of samples is close to a knee point K3.

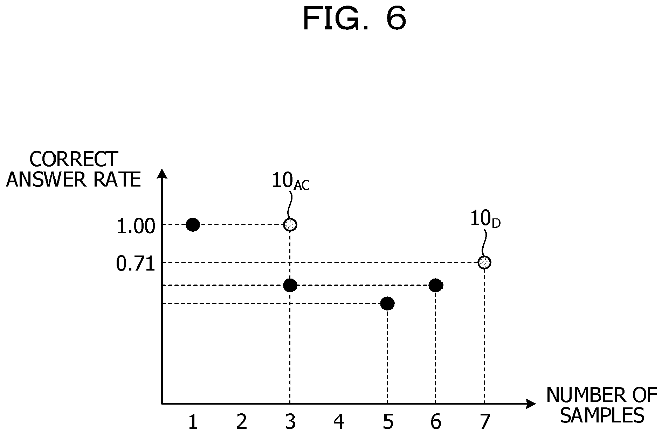

FIG. 6 is a diagram illustrating a relationship between a correct answer rate of a rule corresponding to a designated attribute and the number of samples. The vertical axis of the graph in FIG. 6 is an axis corresponding to the correct answer rate of the rule. The horizontal axis of the graph is an axis corresponding to the number of samples included in the rule. In FIG. 6, a point 10_{AC} indicates the relationship between the number of samples in the rule of the attribute "AC" illustrated in FIG. 4 and the correct answer rate. The point 10_{D} indicates the relationship between the number of samples of the rule of the attribute "D" illustrated in FIG. 4 and the correct answer rate. Since the point 10_{AC} and the point 10_{D} are close to the knee point K3, it may be seen that the rule of the attribute "AC" and the rule of the attribute "D" which are desired by the user may be presented to the user.

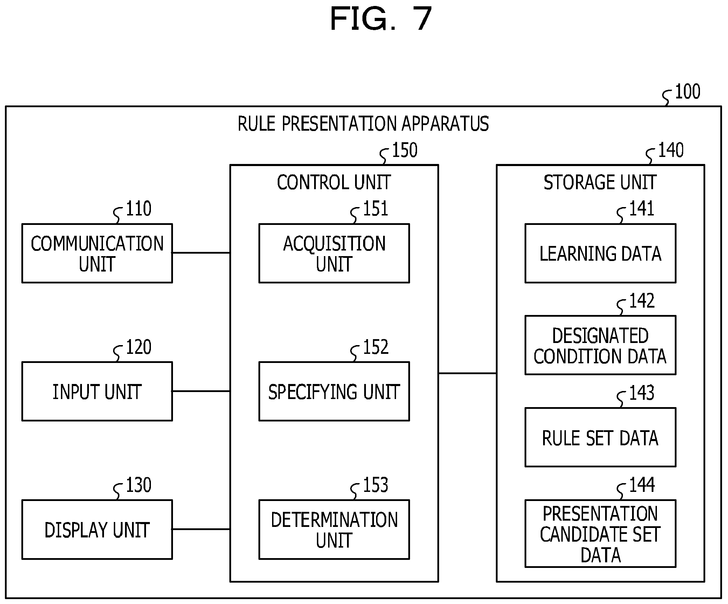

Next, an example of a configuration of the rule presentation apparatus according to this example will be described. FIG. 7 is a functional block diagram illustrating a configuration of the rule presentation apparatus according to this example. As illustrated in FIG. 7, a rule presentation apparatus 100 includes a communication unit 110, an input unit 120, a display unit 130, a storage unit 140, and a control unit 150.

The communication unit 110 is a processing unit that performs data communication with an external device (not illustrated) via a network. The communication unit 110 is an example of a communication device. The control unit 150 described later exchanges data with an external device via the communication unit 110.

The input unit 120 is an input device for inputting various kinds of information to the rule presentation apparatus 100. The input unit 120 corresponds to a keyboard, a mouse, a touch panel, and the like. For example, the user may input designated condition data 142 by operating the input unit 120. The designated condition data 142 is information on the condition of the attribute designated by the user.

The display unit 130 is a display device that displays information output from the control unit 150. For example, the display unit 130 displays information on a rule output from the control unit 150.

The storage unit 140 includes training data 141, designated condition data 142, rule set data 143, and presentation candidate set data 144. The storage unit 140 corresponds to a semiconductor memory element such as a random-access memory (RAM), a read-only memory (ROM), a flash memory, or a storage device such as a hard disk drive (HDD).

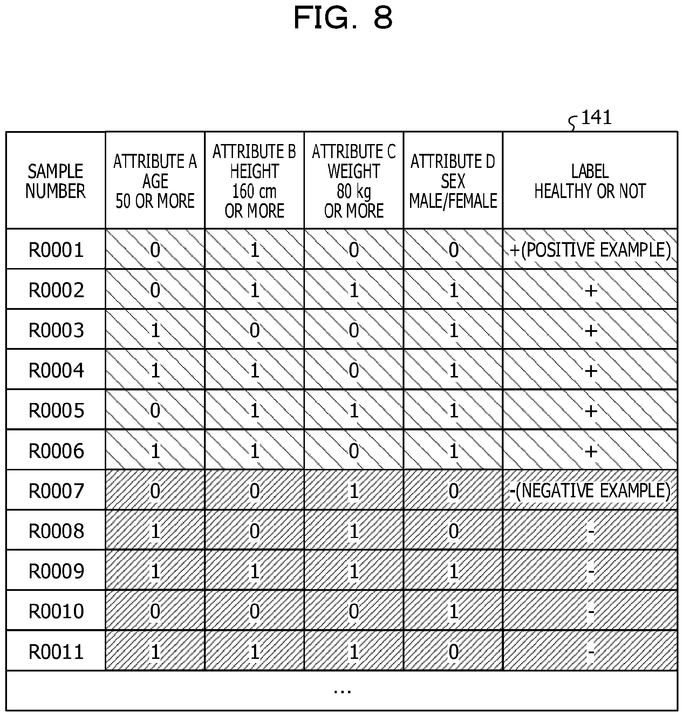

The training data 141 includes a set of rules in which a combination of attributes is associated with a positive example or a negative example. FIG. 8 is a diagram illustrating an example of a data structure of training data. As illustrated in FIG. 8, the training data 141 associates a sample number with each of the attributes A, B, C, and D, and a label. Although the attributes A to D are illustrated in FIG. 8, the training data 141 may have other attributes.

The sample number is information for identifying each sample (record). The attribute A is "indicating whether or not age is 50 (age) or more", and when the age of the subject is 50 or more, a value becomes "1", and when the age of the subject is less than 50, the value becomes "0". The attribute B is "indicating whether or not height is 160 cm or more ", and when the height of the subject is 160 cm or more, the value becomes "1", and when the height of the subject is less than 160 cm, the value becomes "0".

The attribute C is "indicating whether or not weight is 80 kg or more", and when the weight of the subject is 80 kg or more, the value becomes "1", and when the weight of the subject is less than 80 kg, the value becomes "0". The attribute D is "male or female ", and when sex of the subject is male, the value becomes "1", and when sex of the subject is female, the value becomes "0".

The label is "indicating whether or not the value corresponding to each attribute of the sample is "healthy", and the label becomes "+ (positive example)" when it healthy and "- (negative example)" when it is not healthy. For example, in the sample having the sample number "R0001", when the attribute A is "0", the attribute B is "1", the attribute C is "0", and the attribute D is "0", the label is "+". In the sample having the sample number "R0007", when the attribute A is "0", the attribute B is "0", the attribute C is "1", and the attribute D is "0", the label is "-".

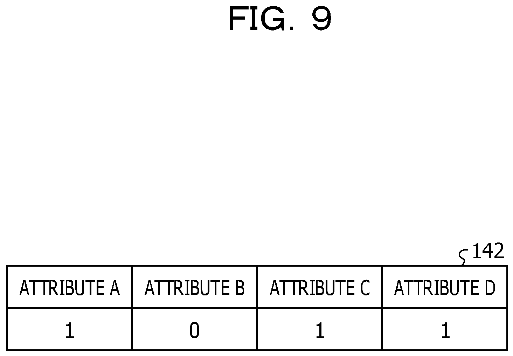

The designated condition data 142 indicates the condition of the attribute designated by the user. FIG. 9 is a diagram illustrating an example of the designated condition data. In the example illustrated in FIG. 9, the attribute A is "1", the attribute B is "0", the attribute C is "1", and the attribute D is "1". Therefore, the condition indicated by the designated condition data 142 indicates "A and notB and C and D".

The rule set data 143 holds data of a plurality of rules specified from the training data 141. The correct answer rate of the rule included in the rule set data 143 is set to be equal to or greater than a threshold value. As an example, the threshold value for the correct answer rate is set to "0.6 (60%)".

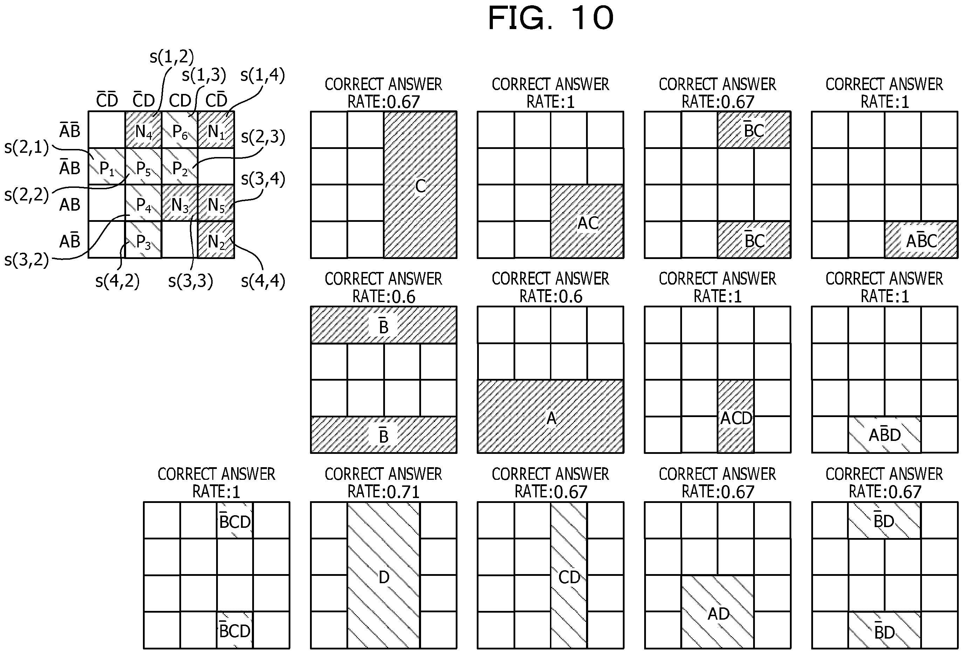

FIG. 10 is a diagram illustrating an example of the rule set data. For example, the Karnaugh map in which P and N are set based on the samples of the training data 141 is illustrated in FIG. 10. For example, for the attributes A to D, the rule whose correct answer rate is equal to or greater than the threshold value among the rules composed of one or more attributes includes the rule (correct answer rate:0.6) of attribute "A", the rule (correct answer rate:0.6) of attribute "notB", the rule (correct answer rate:0.67) of attribute "C", and the rule (correct answer rate:0.71) of attribute "D".

The rule whose correct answer rate is equal to or greater than the threshold value is the rule (correct answer rate:0.67) of attribute "CD", the rule (correct answer rate:0.67) of attribute "notB and C", the rule (correct answer rate:0.67) of attribute "notB and D", the rule (correct answer rate: 1) of attribute "A and C and D", the rule (correct answer rate:1) of attribute "A and notB and C", and the rule (correct answer rate:1) of the attribute "A and notB and D" and the rule (correct answer rate:1) of the attribute "notB and C and D".

The presentation candidate set data 144 holds the data of the rule corresponding to the designated condition data 142 among the rules included in the rule set data 143.

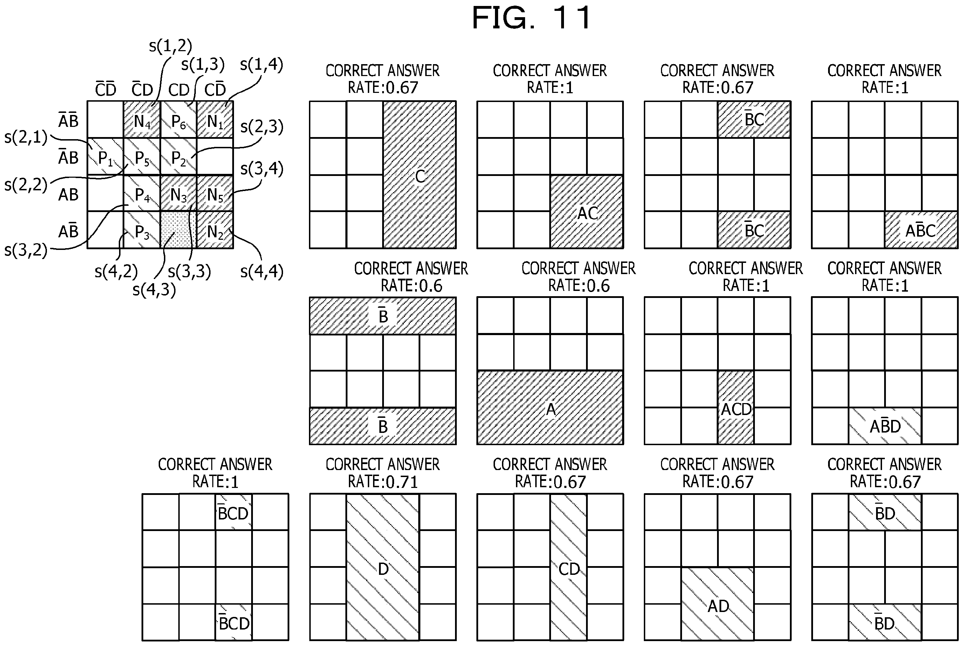

FIG. 11 is a diagram illustrating an example of the presentation candidate set data. For example, the cell of the Karnaugh map corresponding to designated condition data 142 is assumed to be the cell s(4, 3). Among the rules of the rule set data 143, all rules become a rule corresponding to the designated condition data 142.

In FIG. 11, since the correct answer rate is less than the threshold value, although not included in the rule set data 143, description is given by assuming that the correct answer rate of the rule of the attribute "notA" is equal to or greater than the threshold value, and the rule of the attribute "notA" is included in the rule set data 143. In this case, since the rule of the attribute "notA" does not become the rule which corresponds to the designated condition data 142 (does not include the cell s(4, 3)), the rule of the attribute "notA" is excluded from the presentation candidate set data 144.

FIG. 7 will be described again. The control unit 150 includes an acquisition unit 151, a specifying unit 152, and a determination unit 153. The control unit 150 is realized by a central processing unit (CPU), a microprocessor unit (MPU), or the like. The control unit 150 may also be realized by a hardwired logic circuit such as an application-specific integrated circuit (ASIC) or a field-programmable gate array (FPGA).

The acquisition unit 151 is a processing unit that acquires the training data 141 from an external device or the like via a network. When the training data 141 is acquired, the acquisition unit 151 registers the training data 141 in the storage unit 140. The acquisition unit 151 registers the designated condition data 142 in the storage unit 140 when the input of the designated condition data 142 is received by the operation of the input unit 120 by the user.

The specifying unit 152 is a processing unit that specifies a plurality of rules leading to a label of one of the positive and negative examples according to the number of positive examples and the number of negative examples one or more combinations of attributes, based on the training data 141, and registers information of the specified rule in the rule set data 143.

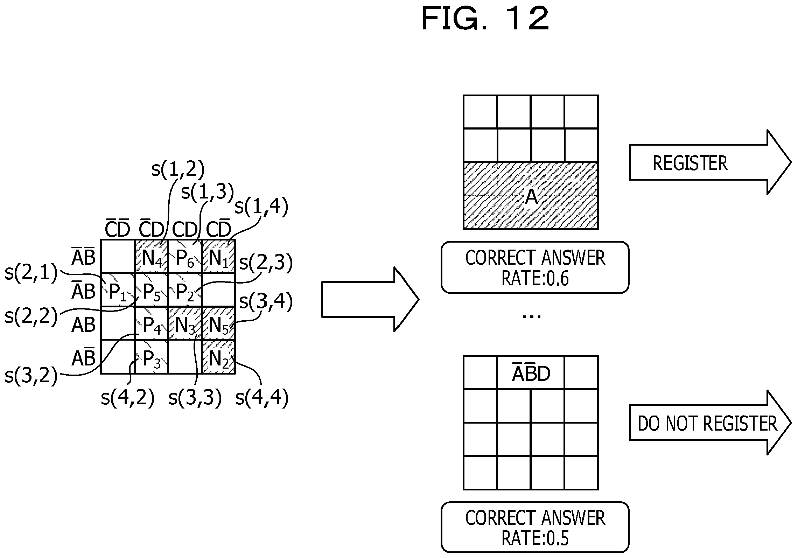

FIG. 12 is a diagram for describing processing of the specifying unit. The specifying unit 152 refers to each sample included in the training data 141, and sets P or N in each cell of the Karnaugh map corresponding to the combination of the attributes of the sample.

In the example illustrated in FIG. 12, the specifying unit 152 sets N₁, N₂, N₃, N₄, and N₅ in the cells s(1, 4), s(4, 4), s(3, 3), s(1, 2), and s(3, 4) of the Karnaugh map, respectively. The rule presentation apparatus sets P₁, P₂, P₃, P₄, and P₅ in the cells s(2, 1), s(2, 3), s(4, 2), s(3, 2), s(2, 2), and s(1, 3) of the Karnaugh map, respectively.

The specifying unit 152 specifies all rules corresponding to the one or more combinations of attributes, calculates the correct answer rate for each specified rule, and specifies a rule whose correct answer rate is equal to or greater than the threshold value as a rule to be registered in the rule set data 143.

The rule of the attribute "A" is a rule that leads to the "negative example". In the rule of the attribute "A", since the number of samples leading to the positive example is two and the number of samples leading to the negative example is three, the correct answer rate is "0.6", which is equal to or greater than the threshold value. Therefore, the specifying unit 152 registers the information of the rule of the attribute "A" in the rule set data 143.

The rule of the attribute "notA and notB and D" is a rule leading to the "positive example" or the "negative example". In the rule of the attribute "notA and notB and D", the number of samples leading to a positive example is one, and the number of samples leading to a negative example is one, and thus the correct answer rate is "0.5", which is less than the threshold value. Therefore, the specifying unit 152 does not register the information of the rule of the attribute "notA and notB and D" in the rule set data 143.

The specifying unit 152 repeatedly executes the processing described above for each rule for one or more combinations of attributes, thereby registering the information on the rule whose correct answer rate is equal to or greater than the threshold value in the rule set data 143.

The determination unit 153 specifies a rule related to the designated condition data 142 among a plurality of rules included in the rule set data 143, and registers information of the specified rule in the presentation candidate set data 144. The determination unit 153 calculates the minimum number of samples for each rule included in the presentation candidate set data 144, and determines the order in which the rules are presented based on the minimum number of samples. The determination unit 153 outputs and displays the rules to the display unit 130 according to the determined order.

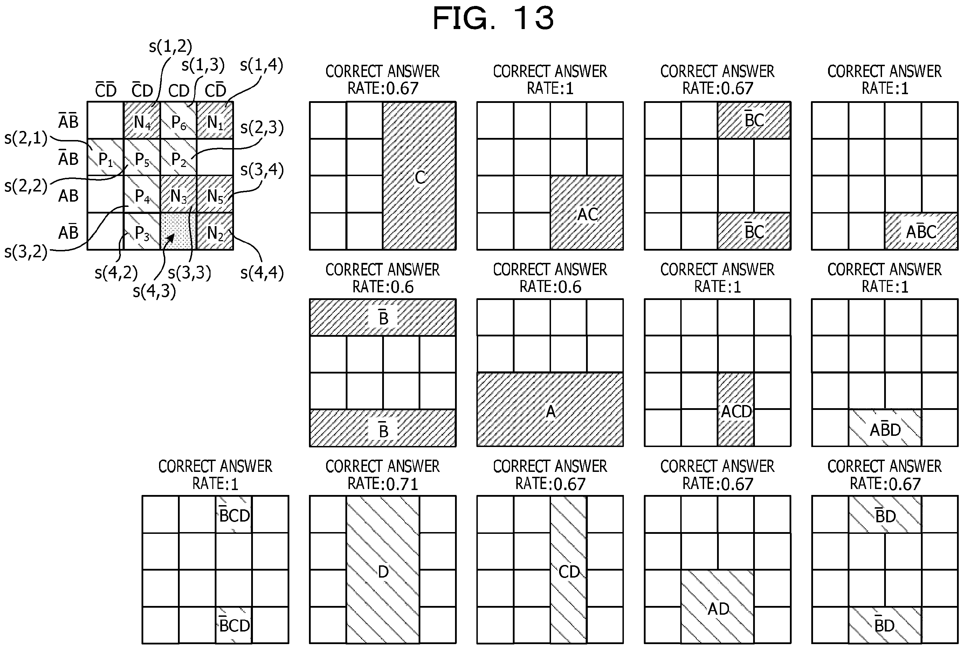

An example of processing in which the determination unit 153 registers rule information related to the designated condition data 142 in the presentation candidate set data 144 will be described. FIG. 13 is a diagram (1) for describing processing of the determination unit. The determination unit 153 compares the attribute of each rule registered in the presentation candidate set data 144 with the attribute corresponding to the designated condition data 142, and specifies the rule related to the combination of the attributes of the designated condition data 142. It is assumed that the condition of the attribute of the designated condition data 142 is "A and notB and C and D".

In the example illustrated in FIG. 13, the cell corresponding to the attribute of the designated condition data 142 is s(4, 3). Therefore, the rules related to the attributes of the designated condition data 142 become the rules of the attributes "C", "A and C", "notB and C", "A and notB and C", "notB", "A", and "A and C and D". The rules related to the attributes of the designated condition data 142 become rules of the attributes "A and notB and D", "notB and C and D", "D", "C and D", "A and D", and "notB and D".

By performing the processing described above, the determination unit 153 specifies a rule related to the designated condition data 142 among the rules registered in the rule set data 143. The determination unit 153 registers information of the rule related to the designated condition data 142 in the presentation candidate set data 144.

Next, an example of processing in which the determination unit 153 calculates the minimum number of samples of each rule of the presentation candidate set data 144 will be described. The determination unit 153 sets a label of the sample corresponding to the designated condition data 142 to a label that is opposite to the label led by the rule, increases the number of samples, and first calculates the number of samples whose the correct answer rate of the rule is less than the threshold value as the minimum number of samples.

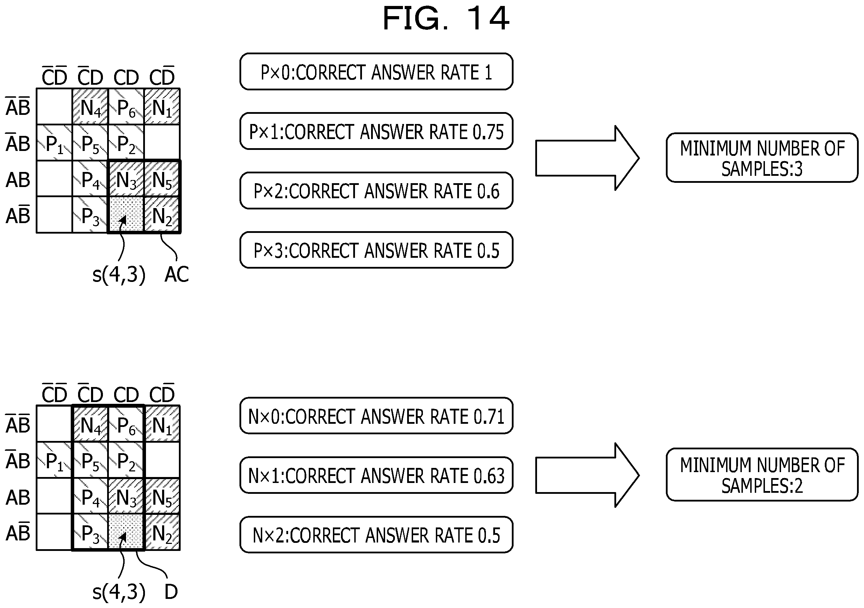

FIG. 14 is a diagram (2) for describing the processing of the determination unit. For example, an example in which the minimum number of samples is calculated using the rule, that leads to the "negative example", of the attribute "A and C" will be described. When the determination unit 153 sets one sample of the attribute "positive example" for the cell s(4, 3) corresponding to the designated condition data 142, the correct rate is 0.75. When the determination unit 153 sets two samples of the attribute "positive example" for the cell s(4, 3) corresponding to the designated condition data 142, the correct rate is 0.6. When the determination unit 153 sets three samples of the attribute "positive example" for the cell s(4, 3) corresponding to the designated condition data 142, the correct rate is 0.5. Therefore, the determination unit 153 calculates the minimum number of samples of the rule, that leads to the "negative example", of the attribute "A and C" as "3".

An example in which the minimum number of samples is calculated using the rule, that leads to the "positive example", of the attribute "D" will be described. When the determination unit 153 sets one sample of the attribute "negative example" for the cell s(4, 3) corresponding to the designated condition data 142, the correct rate is 0.63. When the determination unit 153 sets two samples of the attribute "positive example" for the cell s(4, 3) corresponding to the designated condition data 142, the correct rate is 0.5. Therefore, the determination unit 153 calculates the minimum number of samples of the rule, that leads to the "positive example", of the attribute "D" as "2".

The determination unit 153 repeatedly executes the processing described above for the other rules of the presentation candidate set data 144 to calculate the minimum number of samples of each rule.

The determination unit 153 sorts the rules in descending order of the minimum number of samples based on the minimum number of samples corresponding to each rule registered in the presentation candidate set data 144. The determination unit 153 causes the information of the sorted rule to be displayed on the display unit 130 from the top (in the order of the smallest number of samples).

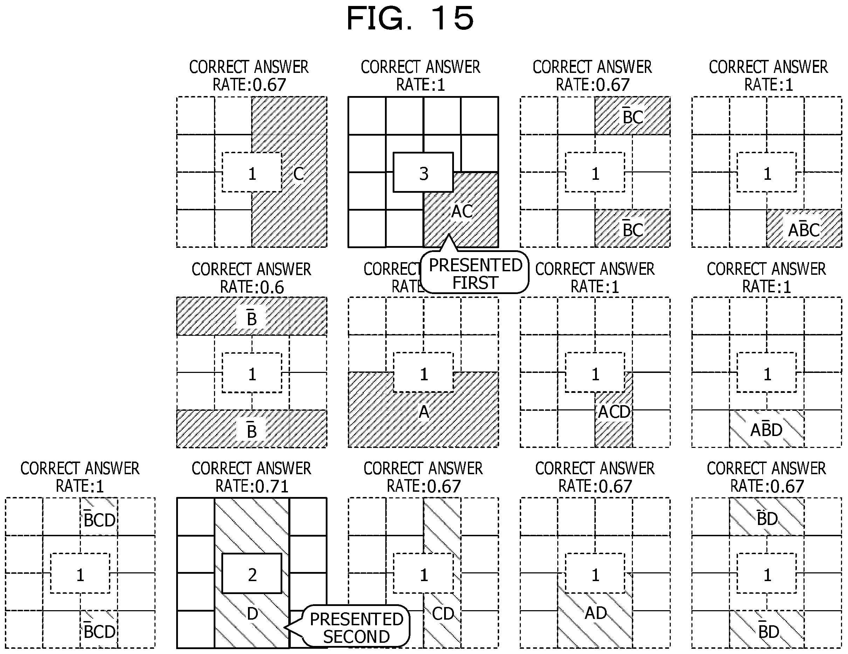

FIG. 15 is a diagram (3) illustrating the processing of the determination unit. For example, as a result of the determination unit 153 sorting in the descending order of the minimum number of samples, the first rule is a rule of the attribute "AC", and the second rule is a rule of the attribute "D". In this case, as illustrated in FIG. 15, the determination unit 153 displays the rule, that leads to the "negative example", of the attribute "AC" first. The determination unit 153 displays the rule, that leads to "positive example", of the attribute "D" second.

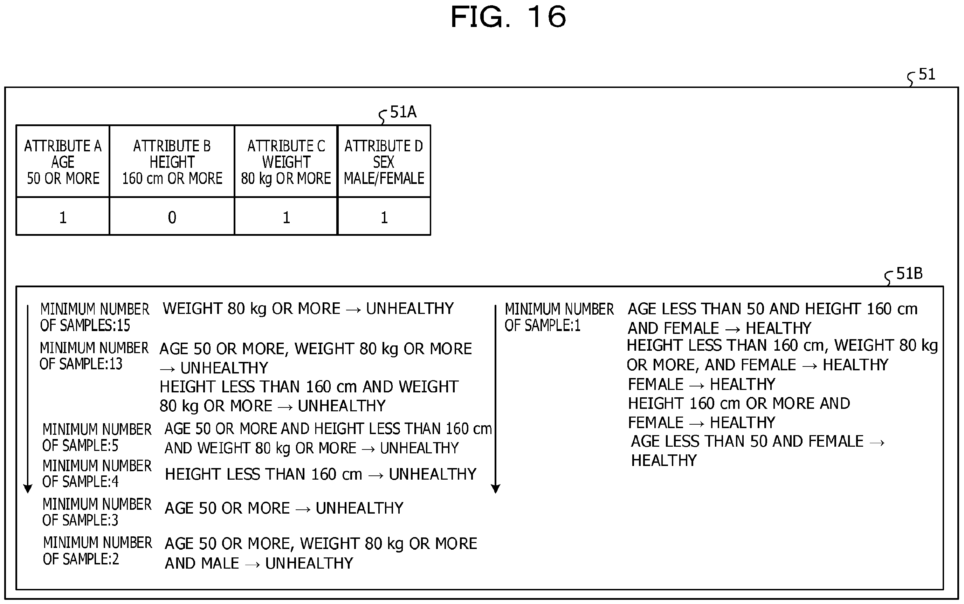

For example, the determination unit 153 may generate screen information for displaying the rule, and output the generated screen information to the display unit 130 to be displayed. FIG. 16 is a diagram illustrating an example of screen information generated by the determination unit. As illustrated in FIG. 16, screen information 51 includes a region 51A and a region 51B.

The region 51A is a region for displaying the designated condition data 142. The region 51B is a region for displaying the rule in the descending order of the minimum number of samples. The determination unit 153 may automatically display the following rules at predetermined time intervals in the region 51B, or may display the rules in order according to the user's operation.

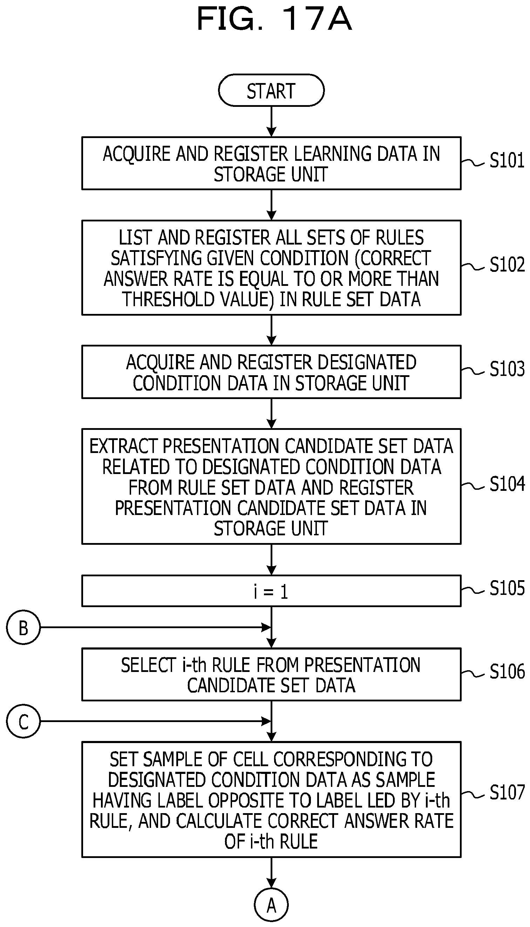

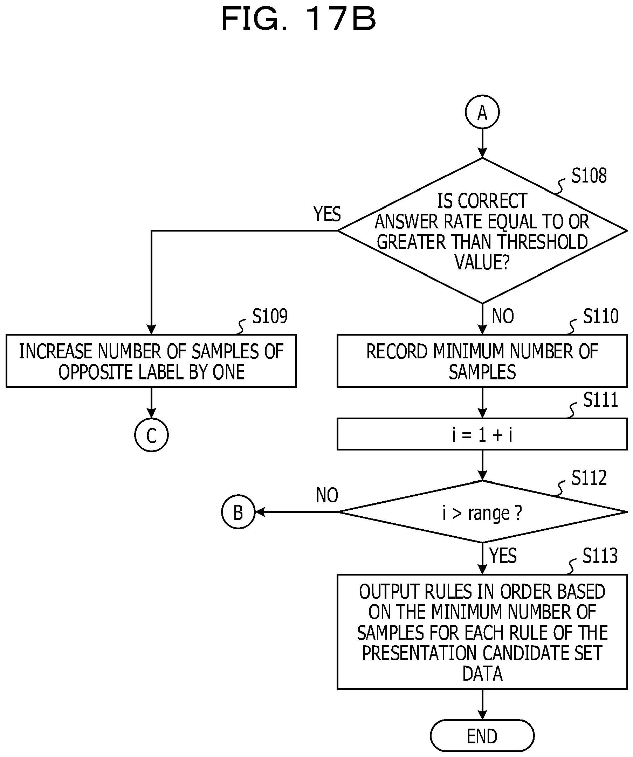

Next, an example of a processing procedure of the rule presentation apparatus 100 according to this example will be described. FIGs. 17A and 17B are a flowchart illustrating the processing procedure of the rule presentation apparatus according to this example. As illustrated in FIGs. 17A and 17B, the acquisition unit 151 of the rule presentation apparatus 100 acquires the training data 141, and registers the training data 141 in the storage unit 140 (step S101). The specifying unit 152 of the rule presentation apparatus 100 enumerates all sets of rules satisfying a given condition (the correct answer rate is greater than or equal to a threshold value) and registers the enumerated sets of rules in the rule set data 43 (step S102).

The determination unit 153 of the rule presentation apparatus 100 acquires the designated condition data 142, and registers the designated condition data in the storage unit 140 (step S103). The determination unit 153 extracts the presentation candidate set data 144 related to the designated condition data 142 from the rule set data 143, and registers the presentation candidate set data in the storage unit 140 (step S104).

The determination unit 153 sets "1" to i (step S105). The determination unit 153 selects the i-th rule from the presentation candidate set data 144 (step S106). The determination unit 153 sets the sample of the cell corresponding to the designated condition data 142 as a sample having a label opposite to the label led by the i-th rule, and calculates the correct answer rate of the i-th rule (step S107).

When the correct answer rate is equal to or greater than the threshold value (Yes in step S108), the determination unit 153 increments the number of samples of the conflicting label by one (step S109), and proceeds to step S107. When the correct answer rate is less than the threshold value (No in step S108), the determination unit 153 proceeds to step S110.

The determination unit 153 records the minimum number of samples (step S110). The determination unit 153 updates the i by adding one to i (step S111). The determination unit 153 determines whether i is larger than a range (the total number of rules of the presentation candidate set data) (step S112). When i is not larger than range (No in step S112), the determination unit 153 proceeds to step S106.

When i is larger than the range (Yes in step S112), the determination unit 153 proceeds to step S113. The determination unit 153 orders the rules of the presentation candidate set data 144 based on the minimum number of samples and outputs the rules (step S113).

Next, effects of the rule presentation apparatus 100 according to this example will be described. The rule presentation apparatus 100 performs rule presentation in the order in which the rule whose label is unlikely to change (the rule with the largest minimum number of samples) is prioritized even if the label corresponding to the designated condition data 142 is opposed to the corresponding rule among a plurality of rules related to the designated condition data 142. As a result, a rule desired by the user may be selected from a plurality of rules corresponding to the designated condition data 142.

The rule with a large minimum number of samples may be said to be a score indicating reliability. Based on the minimum number of samples, the rule presentation apparatus may sequentially present rules in which the trade-off relationship between the correct answer rate and the number of samples is taken into consideration by ordering the rules, even if balance between the height of the correct answer rate of the rule and the number of samples included in the rule is not explicitly designated by the user.

For example, as a simple method of narrowing down a plurality of rules related to the designated condition data 142, it is conceivable to display a rule having a high correct answer rate by paying attention to the correct answer rate. However, a rule with a high correct answer rate tends to be presented with a rule having a small number of samples, and such a rule is susceptible to a change in data and has low reliability.

For example, among the rules described in FIG. 11, the correct answer rate of the rule of the attribute "A and notB and C" is "1", and thus the rule has a high correct answer rate. However, since the sample included in the rule of the attribute "A and notB and C" is one, if the label of the cell s(4, 4) is changed from the "negative example" to the "positive example", the rule is changed from the rule leading to the negative example to the rule leading to the positive example, is easily affected by noise, and have low reliability, and thus, the rule is not a rule desired by the user.

In contrast, in this example, as described with reference to FIG. 6, the rule that is close to the knee point and has high reliability is displayed with priority, and thus a rule desired by the user may be presented with priority.

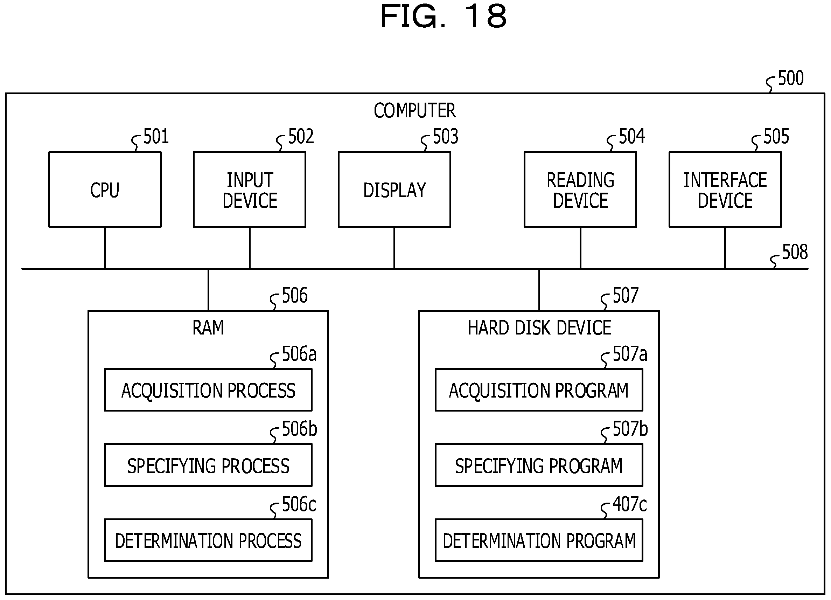

Next, an example of a hardware configuration of a computer that realizes the same function as that of the rule presentation apparatus 100 illustrated in this example will be described. FIG. 18 is a diagram illustrating an example of the hardware configuration of the computer that realizes the same function as that of the rule presentation apparatus according to this example.

As illustrated in FIG. 18, a computer 500 includes a CPU 501 that executes various arithmetic processing, an input device 502 that receives input of data from the user, and a display 503. The computer 500 includes a reading device 504 which reads a program or the like from a recording medium and an interface device 505 which exchanges data with an external device or the like via a wired or wireless network. The computer 500 also includes a RAM 506 that temporarily stores various kinds of information and a hard disk device 507. The respective devices 501 to 507 are coupled to one another by a bus 508.

The hard disk device 507 includes an acquisition program 507a, a specifying program 507b, and a determination program 507c. The CPU 501 reads out the acquisition program 507a, the specifying program 507b, and the determination program 507c, and loads the programs into the RAM 506.

The acquisition program 507a functions as an acquisition process 506a. The specifying program 507b functions as a specifying process 506b. The determination program 507c functions as a determination process 506c.

Processing of the acquisition process 506a corresponds to processing of the acquisition unit 151. Processing of the specifying process 506b corresponds to processing of the specifying unit 152. Processing of the determination process 506c corresponds to processing of the determination unit 153.

The programs 507a to 507c may not be stored in the hard disk device 507 from the beginning. For example, the respective programs may be stored in a "portable physical medium " that is to be inserted in the computer 500, such as a flexible disk (FD), a compact disc read-only memory (CD-ROM), a digital versatile disc (DVD), a magneto-optical disc, or an integrated circuit (IC) card. The computer 500 may read and execute the programs 507a to 507c.

The following appendices are further disclosed with respect to the embodiment including the examples described above.
(Appendix 1) A rule presentation method comprising:
   by a computer,
   acquiring training data that is a set of rules in which a combination of attributes is associated with a positive example or a negative example;
   specifying a plurality of rules that lead to either a positive example or a negative example according to the number of positive examples and the number of negative examples for one or more combinations of attributes, based on the training data;
   acquiring data that has a combination of attributes different from the combination of attributes included in the training data and has unknown labels that designate a positive example or a negative example;
   selecting a rule related to the combination of attributes included in the data from among the plurality of specified rules, setting a label different from a positive example or a negative example led by the selected rule in the data, and specifying the number of samples of the data in which the positive example or the negative example led by the selected rule changes; and
   determining an order of rules to be presented based on the number of samples.
(Appendix 2) The rule presentation method according to appendix 1, wherein in the specifying of the plurality of rules, a larger percentage of a percentage of positive examples or a percentage of negative examples is calculated as a correct answer rate of the rule for a label for one or more combinations of attributes included in the rule, and a plurality of rules whose correct answer rate is equal to or greater than a threshold value are specified.
(Appendix 3) The rule presentation method according to appendix 1 or 2, wherein in the specifying of the number of samples of the data, when the label led to the rule related to the combination of attributes included in the data is a positive example, and a minimum number of samples of the data in which a percentage of positive examples included in the rule is less than a threshold value is specified, a negative example is set as a label of the data.
(Appendix 4) The rule presentation method according to appendix 1, 2, or 3, wherein in the specifying of the number of samples of the data, when the label led to the rule related to a combination of attributes included in the data is a negative example, and a minimum number of samples of the data in which a percentage of negative examples included in the rule is less than a threshold value is specified, a positive example is set as a label of the data.
(Appendix 5) The rule presentation method according to any one of appendices 1 to 4, further comprising: presenting the rule based on the order of the rules determined by the determining.
(Appendix 6) A rule presentation program for causing a computer to execute a process, the process comprising:
   acquiring training data that is a set of rules in which a combination of attributes is associated with a positive example or a negative example;
   specifying a plurality of rules that lead to either a positive example or a negative example according to the number of positive examples and the number of negative examples for one or more combinations of attributes, based on the training data;
   acquiring data that has a combination of attributes different from the combination of attributes included in the training data and has unknown labels that designate a positive example or a negative example;
   selecting a rule related to the combination of attributes included in the data from among the plurality of specified rules, setting a label different from a positive example or a negative example led by the selected rule in the data, and specifying the number of samples of the data in which the positive example or the negative example led by the selected rule changes; and
   determining an order of rules to be presented based on the number of samples.
(Appendix 7) The rule presentation program according to appendix 6, wherein in the specifying of the plurality of rules, a larger percentage of a percentage of positive examples or a percentage of negative examples is calculated as a correct answer rate of the rule for a label for one or more combinations of attributes included in the rule, and a plurality of rules whose correct answer rate is equal to or greater than a threshold value are specified.
(Appendix 8) The rule presentation program according to appendix 6 or 7, wherein in the specifying of the number of samples of the data, when the label led to the rule related to the combination of attributes included in the data is a positive example, and a minimum number of samples of the data in which a percentage of positive examples included in the rule is less than a threshold value is specified, a negative example is set as a label of the data.
(Appendix 9) The rule presentation program according to appendix 6, 7, or 8, wherein in the specifying of the number of samples of the data, when the label led to the rule related to a combination of attributes included in the data is a negative example, and a minimum number of samples of the data in which a percentage of negative examples included in the rule is less than a threshold value is specified, a positive example is set as a label of the data.
(Appendix 10) The rule presentation program according to any one of appendices 6 to 9, the process further comprising: presenting the rule based on the order of the rules determined by the determining.
(Appendix 11) A rule presentation apparatus comprising:
   a specifying unit configured to acquire training data that is a set of rules in which a combination of attributes is associated with a positive example or a negative example and specify a plurality of rules that lead to either a positive example or a negative example according to the number of positive examples and the number of negative examples for one or more combinations of attributes, based on the training data;
   acquiring data that has a combination of attributes different from the combination of attributes included in the training data and has unknown labels that designate a positive example or a negative example;
   a determination unit configured to select a rule related to the combination of attributes included in the data from among the plurality of specified rules, set a label different from a positive example or a negative example led by the selected rule in the data, and specify the number of samples of the data in which the positive example or the negative example led by the selected rule changes, thereby determining an order of rules to be presented based on the number of samples.
(Appendix 12) The rule presentation apparatus according to appendix 11, wherein the specifying unit is configured to, for a label for one or more combinations of attributes included in the rule, calculate a larger percentage of a percentage of positive examples or a percentage of negative examples as the correct answer rate of the rule and specify a plurality of rules whose the correct answer rate is equal to or greater than a threshold value.
(Appendix 13) The rule presentation apparatus according to appendix 11 or 12, wherein the determination unit is configured to set a negative example as a label of the data when a rule related to a combination of attributes included in the data leads to a positive example and specify a minimum number of samples of the data in which the percentage of positive examples included in the rule is less than the threshold value.
(Appendix 14) The rule presentation apparatus according to appendix 11, 12, or 13, wherein the determination unit is configured to set a positive example is as the label of the data when the label led the rule related to the combination of attributes included in the data is a negative example and specify the minimum number of samples of the data in which the percentage of negative examples included in the rule is less than the threshold value.
(Appendix 15) The rule presentation apparatus according to any one of appendices 11 to 14, wherein the determination unit is configured to further present the rule based on the order of the determined rules.

## Claims

1. A rule presentation method executed by a computer, the method comprising:
acquiring training data that is a set of rules in which a combination of attributes is associated with one of a positive example and a negative example;
extracting a plurality of rules that specify one of a positive example and a negative example according to the number of positive examples and the number of negative examples for one or more combinations of attributes, based on the acquired training data;
acquiring first data that has a combination of attributes different from the combination of attributes included in the training data and is not associated with a label that designates the positive example or the negative example;
selecting a rule related to the combination of attributes included in the first data from among the plurality of specified rules;
generating second data in which a label different from the label of the positive example or the negative example specified by the selected rule is associated with the first data;
specifying the number of samples of the first data in which the label of the positive example or the negative example specified by the selected rule changes, based on the generated second data; and
determining an order of rules to be presented based on the number of samples.

2. The rule presentation method according to claim 1, wherein
the specifying process includes:
calculating a larger percentage of a percentage of positive examples or a percentage of negative examples as a correct answer rate of the rule for a label for one or more combinations of attributes included in the rule; and
specifying a plurality of rules whose correct answer rate is equal to or greater than a threshold value.

3. The rule presentation method according to claim 1, wherein
the specifying process includes:
when a rule related to a combination of attributes included in the first data leads to the positive example, setting a negative example as a label of the first data; and
specifying a minimum number of samples of the first data in which a percentage of positive examples included in the rule is less than a threshold value.

4. The rule presentation method according to claim 1, wherein
the specifying process includes:
when a label led to the rule related to a combination of attributes included in the first data is a negative example, setting a positive example as a label of the first data; and
specifying a minimum number of samples of the first data in which a percentage of negative examples included in the rule is less than a threshold value.

5. The rule presentation method according to claim 1, wherein the method further comprising presenting the rule based on the order of the determined rules.

6. A non-transitory computer-readable storage medium storing a program that causes a computer to execute a process, the process comprising:
acquiring training data that is a set of rules in which a combination of attributes is associated with one of a positive example and a negative example;
extracting a plurality of rules that specify one of a positive example and a negative example according to the number of positive examples and the number of negative examples for one or more combinations of attributes, based on the acquired training data;
acquiring first data that has a combination of attributes different from the combination of attributes included in the training data and is not associated with a label that designates the positive example or the negative example;
selecting a rule related to the combination of attributes included in the first data from among the plurality of specified rules;
generating second data in which a label different from the label of the positive example or the negative example specified by the selected rule is associated with the first data;
specifying the number of samples of the first data in which the label of the positive example or the negative example specified by the selected rule changes, based on the generated second data; and
determining an order of rules to be presented based on the number of samples.

7. A rule presentation apparatus, comprising:
an acquisition unit configured to acquiring training data that is a set of rules in which a combination of attributes is associated with one of a positive example and a negative example;
an extract unit configured to extract a plurality of rules that specify one of a positive example and a negative example according to the number of positive examples and the number of negative examples for one or more combinations of attributes, based on the acquired training data;
an acquisition unit configured to acquire first data that has a combination of attributes different from the combination of attributes included in the training data and is not associated with a label that designates the positive example or the negative example;
a select unit configured to selecting a rule related to the combination of attributes included in the first data from among the plurality of specified rules;
a generate unit configured to generate second data in which a label different from the label of the positive example or the negative example specified by the selected rule is associated with the first data;
a specify unit configured to specify the number of samples of the first data in which the label of the positive example or the negative example specified by the selected rule changes, based on the generated second data; and
a determine unit configured to determine an order of rules to be presented based on the number of samples.

8. The rule presentation apparatus according to claim 7, wherein the specify unit configured to:
calculate a larger percentage of a percentage of positive examples or a percentage of negative examples as a correct answer rate of the rule for a label for one or more combinations of attributes included in the rule; and
specify a plurality of rules whose correct answer rate is equal to or greater than a threshold value.

9. The rule presentation apparatus according to claim 7, wherein the specify unit configured to:
when a rule related to a combination of attributes included in the first data leads to the positive example, set a negative example as a label of the first data; and
specify a minimum number of samples of the first data in which a percentage of positive examples included in the rule is less than a threshold value.

10. The rule presentation apparatus according to claim 7, wherein the specify unit configured to:
when a label led to the rule related to a combination of attributes included in the first data is a negative example, set a positive example as a label of the first data; and
specify a minimum number of samples of the first data in which a percentage of negative examples included in the rule is less than a threshold value.

11. The rule presentation apparatus according to claim 7, wherein the apparatus is configured to present the rule based on the order of the determined rules.
